# EUROPEAN PATENT APPLICATION

(11) **EP 4 349 982 A1**
(43) Date of publication of application: **10.04.2024**
(21) Application number: 22199974.1
(22) Date of filing: 06.10.2022
(51) Int. Cl.: C12N 15/01, A61K 35/76, A61P 31/04, C12N 7/02

(54) **IN VITRO METHOD FOR THE PREPARATION OF A LYSIS DEFICIENT VIRUS**

(71) Applicant: Technische Universität München, 80333 München (DE); Falgenhauer, Elisabeth, 81687 Munich (DE)
(72) Inventor: SIMMEL, Friedrich, 80336 München (DE); VOGELE, Kilian, 82399 Raisting (DE); VON SCHÖNBERG, Sophie, 85386 Eching (DE)
(74) Representative: Weickmann & Weickmann PartmbB

(57) **Abstract**

The present invention relates to a method for in vitro provision of lysis deficient viruses, in particular lysis deficient bacteriophages, in a cell-free expression system. Further aspects of the invention relate to a correspondingly provided lysis deficient virus, in particular bacteriophage, and the use thereof.

## Description

The present invention relates to a method for *in vitro* provision of lysis deficient viruses, in particular lysis deficient bacteriophages, in a cell-free expression system. Further aspects of the invention relate to a correspondingly provided lysis deficient virus, in particular bacteriophage, and the use thereof.

Bacteriophages (herein also called "phages") are viruses that specifically infect a host bacterium and proliferate at the expense of this host. They are composed of proteins that encapsulate a DNA or RNA genome. Bacteriophages replicate within a host bacterium by injecting their viral genetic material into the host cell effectively taking over the cells functions for the production of progeny bacteriophage leading to the rupture of the cell wall and subsequent bacterial cell death.

The biotechnological fields of application of bacteriophages are very broad and extend from evolution-based selection methods, like the evolutionary improvement of the activity of enzymes and other proteins, to phage display by which biological drug substances, e.g. therapeutic antibodies, could be generated and optimized, to the application of the bacteriophages themselves as a substitute for antibiotics, i.e., bacteriophage therapy.

The latter approach that has been applied for a longer time to fight microbial infections gets more and more attention as the number and spread of multiresistant bacteria strains increases strongly worldwide.

Methicillin-resistant *Staphylococcus aureus* (MRSA) bacteria, for example, is an increasingly common form of infection, often acquired through transmission in hospitals. MRSA infections are extremely difficult to treat using conventional antibiotics. The development of novel antibiotics is significantly slower than this development. So far, bacteriophage therapy is based on the natural characteristics of bacteriophages to specifically affect bacteria, in particular pathogenic bacteria, and kill by lysis. For most phages, active and timed lysis involves three steps, i.e., destruction of the inner bacterial membrane, dissolution of the peptidoglycan layer, and finally disruption of the outer bacterial membrane.

Among the components of the bacterial cell walls releases after lysis are the so-called endotoxins, such as lipopolysaccharides. Released endotoxins activate diverse immune cells.

The administration of many phages at the same time during bacteriophage therapy results in simultaneous bacterial lysis and, consequently, high endotoxin release. The simultaneous release of large amounts of endotoxins by simultaneous lysis results in a strong immune system response, triggering inflammatory processes, which can lead to severe medical conditions such as septic shock, disseminated intravascular coagulation or organ failure.

Further, the development and production of phage-based therapeutics and diagnostics is currently still hampered by the difficulty of finding a simple and safe method to produce bacteriophages. According to the state of the art, bacteriophages are produced by cultivation with the appropriate bacterium/pathogen (Pirnay et al.). This requires compliance with the appropriate safety regulations for the respective bacteria, as well as the ability to culture them. For dangerous pathogens, it thus complicates the handling, which is only possible by specially trained personnel in special facilities and thus also increases the cost factor.

Thus, it is the object of the present invention to provide a simple and safe method for the provision of a virus, in particular bacteriophage, which after administration does not trigger a strong immune system response by endotoxin release.

A first aspect of the invention is an *in vitro* method for the provision of a lysis deficient virus, in particular a lysis deficient bacteriophage, comprising the steps
(a) providing a virus nucleic acid, in particular bacteriophage nucleic acid, comprising a holin, endolysin and/or spanin knockout mutation,
(b) providing a cell-free expression system,
(c) combining the virus nucleic acid, in particular the bacteriophage nucleic acid, and the cell-free expression system, and
(d) incubating the combined virus nucleic acid, in particular bacteriophage nucleic acid, and the cell-free expression system under conditions suitable for expression and self-assembly of the lysis deficient virus, in particular lysis deficient bacteriophage, and
(e) isolating the self-assembled lysis deficient virus, in particular bacteriophage.

A bacteriophage according to the present invention is a virus that specifically infects and replicates within host bacteria and/or archea. Lysis deficient bacteriophages encompass proteins that encapsulate a DNA or RNA genome with a holin, endolysin and/or spanin knockout. The terms "bacteriophage" and "phage" may be applied interchangeably herein.

Endolysin and holin are proteins which are essential for host bacteria lysis by bacteriophages. Other proteins, called spanins, are involved in the lysis process in gram-negative host bacteria. Knockouts of the three enzymes holin, endolysin, and spanin provide lysis-deficient viruses, in particular lysis deficient bacteriophages.

The terms holin, endolysin and spanin are well known to the skilled person. Commonly known sequence numbers for holin, endolysin and spanin are, e.g., in T7 *E.coli* phage for holin P03802 (HOLIN_BPT7), 67 amino acids, for endolysin Q3V7D1 (Q3V7D1_BPT7), 151 amino acids, and for spanin P03803 (SPAN1_BPT7), 143 amino acids.

Holins are small membrane proteins that accumulate in the membrane until, at a specific time programmed into the holin gene, the membrane suddenly becomes permeable to the fully folded endolysins. By accumulating in the host cytoplasmic membrane, holins control the timing of lysis through reaching a critical concentration, thereby forming nanometer-scale holes. These allow soluble endolysin to escape from cytoplasm and to degrade the peptidoglycan layer, following outer membrane disruption by spanin. Via the aggregation into oligomers, holins render the host bacterium cytoplasmic membrane permeable, leading to a loss of polarization. Subsequently, endolysins reach the peptidoglycan layer through the holes in the cell membrane. Endolysins represent muralytic enzymes that degrade the cell wall.

Spanins are often used for the final lysis in gram-negative bacteria. Spanins bridge the inner and outer membranes in form of either unimolecular or heterodimer molecules. After cell wall degradation by endolysin, spanin complexes diffuse and aggregate. The release of sufficient spanin complexes in the degraded region of the peptidoglycan layer results in a lytic blowout.

Lysis deficient viruses, on particular bacteriophages, do not have a lysogenic life cycle and offer a plethora of starting points to prevent endotoxin release during phage therapy. Thus, by providing holin, endolysin and/or spanin knockout virus mutants, in particular bacteriophage mutants, simultaneous bacterial lysis and release of endotoxins can be avoided, preventing strong immune response during phage therapy.

Such holin, endolysin and/or spanin knockout virus or bacteriophages still specifically kill bacteria, e.g. by degrading the bacterial genome, without lysing them. Finally, the genome-less bacteria are slowly degraded by the immune system.

The term "gene knockout" is well known to a skilled person. It is a genetic technique in which one of an organism's genes is made inoperative/inactive ("knocked out" of the organism). However, knockout can also refer to the gene that is knocked out or the organism that carries the gene knockout. It is possible to knock out one, two or even more genes simultaneously. Methods for providing such gene knockouts are well known to those skilled in the art. Knockouts are accomplished through a variety of methods, for example homologous recombination, gene silencing, introduction of point mutations, conditional knockout, gene-editing using gene-editing nucleases such as site-specific nucleases, zinc finger nucleases, transcription activator-like effector nucleases, and CRISPR/Cas9, which relies on frameshifts caused by insertion/deletion, and alteration of the DNA by deletion/insertion. All methods have in common that they inhibit the normal function of the gene and thus inactivate the changed gene, i.e., generate a knockout.

Nonetheless, the production of holin, endolysin and/or spanin knockout bacteriophage mutants, i.e., lysis deficient viruses/bacteriophages, applying conventional methods for phage provision is difficult. The development of phage-based therapeutics and diagnostics is, however, prevented by problems in the production and/or genetic modification of bacteriophages. The complexity of the modification of bacteriophages is mainly due to the difficulty of changing the genome of bacteriophages.

Conventionally, bacteriophages are produced by *in vivo* cultivation with the appropriate bacterium and released by lysis at the end of the maturation phase. Since lysis deficient bacteriophages remain in the bacteria, the bacteria have to be digested separately, which, depending on the host bacterium, is difficult to perform without significantly reducing the titer of the bacteriophages. Also, the purification of bacteriophages produced by *in vivo* cultivation as such has proven to be difficult and complex.

The virus nucleic acid, in particular bacteriophage nucleic acid, comprising a holin, endolysin and/or spanin knockout mutation may be provided from any suitable source. The nucleic acids may be provided, for example, in the form of YACs (Yeast Artificial Chromosome), BACs (Bacterial Artificial Chromosome), PACs (P1 Artificial Chromosome) or MACs (Mammalian Artificial Chromosome).

According to a preferred embodiment step (a) for providing the virus nucleic acid, in particular bacteriophage nucleic acid, comprising a holin, endolysin and/or spanin knockout mutation may comprise *in vitro* amplification of a virus nucleic acid, in particular a bacteriophage nucleic acid such as a bacteriophage genome, encoding the lysis deficient virus, in particular lysis deficient bacteriophage, may preferably comprise the steps of
(i) providing a virus nucleic acid template, in particular a bacteriophage nucleic acid template,
(ii) sensitizing, in particular methylating, the virus nucleic acid template, in particular the bacteriophage nucleic acid template, for modification specific restriction enzymes,
(iii) amplification of the virus nucleic acid template, in particular the bacteriophage nucleic acid template, using polymerase chain reaction to amplify and provide different nucleic acid segments thereby introducing nucleic acid mutations to provide holin, endolysin and/or spanin knockout mutants,
(iv) wherein the primers used for at least one 5'-end of the virus nucleic acid template, in particular the bacteriophage nucleic acid template, optionally introduce nucleic acid exonuclease degradation blocking modifications, in particular phosphorothioate bonds, between the first nucleotides at the at least one 5'-end of the corresponding nucleic acid segments,
(v) digesting the methylated virus nucleic acid template, in particular the bacteriophage nucleic acid template,
(vi) recessing by an 5'-3'-directional exonuclease, in particular T5 exonuclease, to provide sticky end nucleic acid segments,
(vii) annealing of the sticky nucleic acid segments and
(viii) ligating of the annealed nucleic segments to provide amplified linear virus nucleic acids, in particular bacteriophage nucleic acids.

The virus nucleic acid, in particular bacteriophage nucleic acid, comprising a holin, endolysin and/or spanin knockout mutation may comprise a holin and an endolysin knockout mutation, a holin and a spanin knockout mutation, a spanin and an endolysin knockout mutation as well as a holin, a spanin and an endolysin knockout mutation. Spanin knockout mutations are particularly preferred if the bacterium to be targeted by the provided bacteriophage is a gram-negative bacterium.

According to the present invention, it is possible to carry out the production of lysis deficient synthetic viruses, in particular lysis deficient bacteriophages, completely in *vitro,* i.e. in a completely cell-free system.

The process is based on a combination of *in vitro* methods for reproducing nucleic acids, in particular DNA, wherein the nucleic acid to be amplified encodes the lysis deficient virus or lysis deficient bacteriophage, as well as completely producing lysis deficient bacteriophages or viruses *in vitro* by means of cell-free protein expression in a cell extract or lysate.

According to a preferred embodiment of the present invention the virus nucleic acid to be amplified in step (a'), i.e. the template, may be a bacteriophage nucleic acid and, in particular, a bacteriophage genome encoding the lysis deficient bacteriophage. A genome to be amplified according to the present invention is preferably a linear genome. The amplified lysis deficient bacteriophage genome may be further modified.

The nucleic acid provided in step (a) or the nucleic acid template to be amplified in step (a') may be selected from the group consisting of DNA, RNA, in particular double stranded DNA or double stranded RNA, and any variants thereof. "Variants" in particular encompasses conservatively modified nucleic acids (such as degenerate codon substitutions) and complementary sequences.

Bacteriophages are classified by the international Committee on Taxonomy of Viruses according to morphology and nucleic acid, including dsDNA-phages, ssDNA-phages, dsRNA-phages as well as ssRNA-phages. The present invention is not limited to a certain class of phages. However, dsDNA-phage are in particular preferred. Preferred dsDNA-phages comprise *Ackermannviridae, Autographiviridae, Chaseviridae, Demerecviridae, Drexlerviridae, Herelleviridae, Myoviridae, Podoviridae, Siphoviridae, Tectiviridae, Corticoviridae, Plasmaviridae, Lipothrixviridae, Rudiviridae, Fuselloviridae, Halspiviridae, Guttaviridae, Bicaudaviridae, Thaspiviri*dae and *Autolykiviridae.* According to the present invention, a bacteriophage is a preferred embodiment of a virus.

According to step (a) or (a') of the inventive method, the virus or bacteriophage nucleic acid to be expressed or the virus or bacteriophage nucleic acid template to be amplified is provided. Methods for providing such nucleic acids or nucleic acid templates are well established in the art and well known to the skilled person.

During amplification step (a'), a bacteriophage nucleic acid template is modified to produce a knockout variant of a phage that can no longer express holin, endoslysin or spanin. Hence, the nucleic acid template, in particular the bacteriophage nucleic acid template is first sensitized for modification specific restriction (digestion) enzymes, in particular methylated, according to step (ii) to make it distinguishable from any new product, i.e. the amplified modified nucleic acid segment. This step efficiently minimizes the number of false positive viruses for modification, in particular false-positive phages for modification that otherwise would be generated based on the unmodified DNA. The respective enzymes for methylation are known to a person skilled in the art and comprise, for example, preferably Dam-methylase.

Amplifying a nucleic acid template by polymerase chain reaction (PCR) according to step (iii) is, in general, an established technology.

Depending on the template, respective primers have to be provided. Primer design procedures are well known to those skilled in the art. Various biotechnology companies offer tailor-made primers for all molecular biology applications and/or template-specific requirements. In principle, the primers can be designed so that the template nucleic acid, in particular the genome to be amplified, is completely amplified and/or that the individual nucleic acid segments to be provided are substantially of comparable nucleic acid lengths. Of course, the provided individual nucleic acid segments can be also of different nucleic acid length.

Nucleic acid mutations providing holin, endolysin and/or spanin knockout mutants may be introduced by deletion, insertion and/or point mutation of the template nucleic acids, wherein the nucleic acid mutations preferably provide holin and endolysin knockout mutants.

The terms "deletion", "insertion", and "point mutation" as well as the corresponding experimental methods are well known to a skilled person. Deletion of genes changes the DNA sequence by removing at least one nucleotide in a gene. Small deletions remove one or a few nucleotides within a gene, while larger deletions can remove an entire gene or several neighboring genes. Types of deletion include terminal deletion, intercalary/interstitial deletion, and microdeletion. Gene insertion is the addition of one or more genes into a DNA sequence, a technique that has been traditionally performed with plasmid DNA or integrating viral vectors. A point mutation is a genetic mutation where single or more nucleotide bases are substituted, inserted or deleted from a DNA or RNAsequence.

Preferably, nucleic acid segments have a size in the range of about 5 kbp to about 30 kbp, preferably about 5 kbp to about 25 kbp, about 5 kbp to about 15 kbp, particularly preferred about 10 kbp.

Primers used according to step (iv) for at least one 5'-end of the template nucleic acid introduce nucleic acid exonuclease degradation blocking modifications, in particular backbone modifications. It is preferred that the primers introduce corresponding modifications at one 5'-end of the template.

According to the present invention, DNA exonuclease degradation blocking modifications, in particular backbone modifications, are modifications that lead to nuclease resistance, in particular to resistance against 5'-exonuclease. In general, such modifications can be differentiated between backbone, terminal and internal base modifications. Only few options of backbone modifications are available: phosphorothioate bonds (PTO bonds), locked nucleic acids (LNAs) and/or incorporation of aliphatic spacers are examples of backbone modifications. Terminal modifications at the 5'-end, most commonly fluorescent dyes or linker groups and/or internal base modifications at the 2'O exemplify terminal and internal base modifications, in particular in RNAs and/or mixed oligonucleotides. The use of backbone modifications is preferred.

Thus, preferred examples of backbone modifications according to the present invention include phosphorothioate bonds (PTO bonds).

It is preferable that the first 1 to 15, more preferable 1 to 10, more preferable 1 to 8 and even more preferably 1 to 5 nucleotides of the first 5'-nucleotides and/or corresponding bonds of the respective primer are modified. According to other preferred embodiments of the invention 5-20, more preferably 5-18, more preferably 5-15 of the first 20 5'-nucleotides and/or corresponding bonds of the respective primer are modified or 5-15, more preferably 5-12 of the first 15 5'-nucleotides and/or corresponding bonds of the respective primer.

An insertion of DNA exonuclease degradation blocking modifications, such as by applying a primer comprising the respective oligonucleotides, prevents exonuclease degradation at the respective 5'-end. By introduction of such modifications undesired circulation of linear DNA fragments, in particular during litigation step (viii), can be prevented.

For that purpose, the phosphodiester bonds of the first 5' nucleotides of the primer may be substituted with phosphorothioate bonds (PTO bonds). During PCR they are inserted into the DNA fragments and prevent the applied 5'-exonuclease, in particular an applied T5 exonuclease, from producing the overhangs at those positions necessary for ligation. This is a surprising result since the 5'-exonucleases are normally not stopped by the thiophosphates in the backbone of a DNA.

Thus, an especially preferred embodiment of a DNA exonuclease degradation blocking modification, i.e. circulation-blocking backbone modification is based on the introduction of phosphorothioate, i.e. the introduction of phosphorothioate bonds (PTO bonds). A DNA phosphorothioate modification is a modification on the DNA backbone, in which a non-bridging oxygen atom is replaced with a sulfur atom. The respective phosphorothioate modificated DNA may be provided by the use of phosphorothioate comprising primers during the PCR reaction. Phosphorothioate comprising primers may be provided by using phosphorothioate oligonucleotides replacing non-bridging oxygen atom on the DNA backbone with a sulfur atom when generating and/or providing respective primers.

It is preferable that the first 1 to 20, more preferably 8 to 20, more preferable 10 to 20 and even more preferably 10 to 15 phosphodiester bonds of the first 5'-nucleotides of the respective primer may be substituted with phosphorothioate bonds. According to other preferred embodiments of the invention 5-20, more preferably 5-18, more preferably 8-15 phosphodiester bonds of the first 20 5'-nucleotides of the respective primer may be substituted, or 8-15, more preferably 8-12 phosphodiester bonds of the first 15 5'-nucleotides of the respective primer.

After amplification of the template DNA via PCR in step (iii) and step (iv) the methylated template DNA is digested by a restriction enzyme sensitive to methylation according to step (v). The respective enzymes are known to a person skilled in the art and comprise, for example, preferably Dpnl.

By this method any false-positive viruses for modification, in particular false-positive phages for modification, can be reduced to a minimum that would otherwise be generated based on the unmodified DNA. The method can be applied for any suitable modification such as the generation of holin, endolysin, and/or spanin knockouts by deletion, insertion and/or mutation, in particular point mutation of the template nucleic acids.

Accordingly, to a preferred embodiment the inventive method comprises a step of sensitizing, in particular methylating, the virus nucleic acid template, in particular the bacteriophage nucleic acid template, for modification specific restriction enzymes, before amplification step (iii) and step (iv), and a step of digesting the sensitized, in particular methylated, nucleic acid template after amplification step (iii) and step (iv). Suitable modifications for sensitizing the template for modification specific restriction (digestion) enzymes are known to the person skilled in the art. Suitable modifications comprise 5-methylcytosine (5mC), 5-hydroxymethylcytosine (5hmC), 5-formyl-cytosine (5fC), 5-carboxylcytosine (5caC), N6-methadenine (6mA) and, in particular, any kind of suitable methylation.

The 5'-3'-directional exonuclease according to step (vi) can be any enzyme that works by cleaving nucleotides from the 5'-end (exo) of the amplified nucleic acid template of step (iii) and step (iv). A hydrolyzing reaction that breaks phosphodiester bonds at the 5'-end occurs. The terms 5'-3'-directional exonuclease and 5'-exonuclease may be used interchangeably herein.

By action of the 5'-exonuclease 3'-sticky-end nucleic acid segments are provided. A 3'-sticky-end nucleic acid segment shows a 3'-nucleotide overhang.

Examples of suitable 5'-exonucleases are T7-exonuclease, Exonuclease VIII (truncated), Lambda Exonuclease, T5-exonuclease and any mixtures thereof. An 5'-exonuclease which is particularly preferred is T5-exonuclease.

If during amplification step (iii) and step (iv) backbone modifications are used, the use of T5-exonuclease and/or Lambda exonuclease is preferred; if nucleic acid base modifications are used, the use of T7 exonuclease is preferred.

The provided sticky end nucleic acid segments are annealed in step (vii). Annealing conditions and in particular the temperature depend on the template to be amplified and can in turn be determined by the person skilled in the art. The stoichiometric ratio of the sticky-end nucleic acid segments to be annealed is preferably from 1:1 to 1:5 and in particular about 1:1.

After annealing according to step (vii), a polymerase, in particular a DNA polymerase such as Taq-polymerase, may fill nucleotide gaps between the annealed nucleic acid segments with free nucleotides before ligating according to step (viii).

The annealed sticky-end nucleic acid segments are ligated according to step (viii) to provide the amplified linear nucleic acid, in particular bacteriophage nucleic acids. In this step, the 3'-hydroxy end of one nucleic acid segment is linked to the 5'-phosphate end of another nucleic acid segment with the help of the enzyme ligase by forming a phosphodiester bond.

According to a preferred embodiment steps (i) to (viii) can be carried out in a single tube reaction without isolation and/or purification of any intermediates, in particular using a one-step mastermix of enzymes. Thus, a combination of PCR amplification of single genome segments and a subsequent ligation using so-called "Gibson assembly" is performed.

The method described above comprising step (i) to step (viii) is based on the Gibson assembly, an *in vitro* enzymatic reaction which is known in the art to ligate two or more DNA-fragments that have overlapping sequences at their ends. The basis of the Gibson assembly method is the production of DNA fragments by PCR with the primer being selected such that the DNA fragments have overlapping sequences. Then the fragments are combined by the combined activity of three enzymes. An exonuclease, preferably T5 exonuclease, generates 3'-overhangs that hybridize with the complementary strands of the complementary sequences of the adjacent strand. A DNA polymerase fills the gaps that occurred and a ligase binds the DNA covalently. The method can also be used for site-directed mutagenesis to incorporate site-specific mutations such as insertions, deletions and/or point mutations for the production of lysis deficient viruses, in particular lysis deficient bacteriophages.

However, the known Gibson assembly method bears a problem. Many phage genomes bear identical sequences on both ends of the genome that are combined by a Gibson assembly so that a circular genome is generated and a reproduction of the linear DNA *in vitro* is no longer possible. In order to prevent the ends of the genome, which often have overlapping sequences, from circulating the primers for the ends of the genome are modified.

This problem is, in particular, solved by the present invention by applying primers at at least one 5'-end of the virus or bacteriophage nucleic acid template, which insert exonuclease degradation blocking modifications.

According to an especially preferred embodiment, the virus or bacteriophage nucleic acid or genome is provided by the method herein described above.

A "cell-free expression system" as understood herein under step (b) is host independent, preferably selected from cell lysates or artificial expression systems, and comprises a complete transcription and translation machinery for transcription and expression of a virus or bacteriophage nucleic acid, in particular a bacteriophage genome. The term "cell free" is understood by the person skilled in the art and refers to "substantially free of".

Within a cell lysate, the protein synthesis can be performed using the transcription and translation apparatus of lysed cells. The cell lysate is free of host DNA after purification, i.e., nucleic acids, in particular DNA, and/or membranes from the cells of which it is derived, and allows expression of the desired protein by external addition of DNA, in particular an amplified bacteriophage genome, encoding the lysis deficient virus, in particular lysis deficient bacteriophage, as herein described. Using such a cell lysate, it is possible to synthesize several proteins or metabolites at the same time.

Combining and incubating a cell free expression system for the production of lysis deficient bacteriophages with the amplified virus nucleic acid, in particular the amplified bacteriophage nucleic acid, provided by step (a) results in exclusive transcription, translation, expression, and self-assembly of the added amplified bacteriophage genome, encoding the lysis deficient virus, according to step (c) and step (d). After bacteriophage self-assembly in the cell free system according to step (d), the resulting lysis deficient bacteriophages are complete and active, i.e. they are identical in form and function to wild-type phages, except for the inserted modification, in particular lysis deficiency. As last step, the self-assembled lysis deficient viruses, in particular bacteriophages, are isolated according to step (e).

Using the cell free method according to the present invention, a lysis deficient bacteriophage solution is generated without any prophages. The skilled person understands under the term "prophage" phage DNA, which has been incorporated into the genome of bacteria. Prophages can be indirect triggers of various human diseases, such as *Streptococcus pyogenes* (scarlet fever), *Clostridium botulinum* (botulism) and *Vibrio cholerae* (cholera).

"Cell lysate" according to the present invention refers to a fluid comprising the components of cells from which it is derived after lysis. Lysis methods are known to the person skilled in the art and break down the membrane of a cell, for example, by viral, enzymatic, or osmotic mechanisms that compromise its integrity. Such cell lysate is essentially void of intact cells, i.e. cell-free. The terms "cell lysate" and "cell extract" can be used herein interchangeably.

Cell lysates used according to the present invention may be derived from microorganisms, in particular bacteria such as pathogenic bacteria and E. *coli,* yeast, insects, mammals and/or plants, in particular such as wheat or rice, or may be even artificial. "Microorganism" refers to a bacterium or an archaeon. Preferably, the microorganism is a bacterium.

A number of cell-free, artificial expression systems are known to a person skilled in the art und may be applied according to the present invention. For example, the artificial expression system "PURE" consists of several isolated proteins (Shimizu et al.) while untreated cell lysates (crude extracts) such as of E. *coli* include almost all intracellular proteins, even those that are not necessary for expression. In such untreated cell extracts, it has already been shown that it is possible to express infectious wild-type bacteriophages (Shin et al. 2012), as well as proteins (Garamella et al. 2016).

The use of E. *coli* lysate, in particular crude E. *coli* lysate, is a further preferred embodiment. A preferred example of crude E. *coli* lysate is *E.coli* S30 cell extract produced by a method based on the protocol described in Z.Z Sun et al. (Z.Z. Sun, C.A. Hayes, J. Shin, F. Caschera, R.M. Murray, V. Noireaux., J Vis Exp. 2013, 79, 50762).

The cell-free lysate to be used is derived from a cell or microorganism, which is different to the natural host of the virus or bacteriophage. Thus, at least one protein and/or a nucleic acid encoding a protein to enhance or otherwise improve the expression and/or self-assembly preferably selected from the group comprising co-factors, chaperons, polymerases, transcription regulatory factors, and/or any mixtures thereof, is added during step (b), (c) and/or (d). Such factors may be added as proteins and/or as nucleic acid encoding a respective protein. At least one bacteriophage-host specific expression factor and/or a nucleic acid encoding the at least one bacteriophage-host specific expression factor, preferably a transcription factor, may be added.

According to one embodiment of the invention, at least one nucleotide sequence encoding the at least one bacteriophage-host specific expression factor or any other protein preferably selected from the group comprising co-factors, chaperons, polymerases, transcription regulatory factors is cloned into the cell strain used to provide the cell lysate.

The method can also be used to produce a further modified lysis deficient bacteriophage wherein, beside holin, spanin and/or endolysin, at least one further protein of interest is modified or added. The modified or additional protein of the bacteriophage can be provided by adding that protein to the cell-free expression system and/or by adding a nucleic acid encoding that protein. This protein itself can be of natural amino acid sequence or artificially modified for optimization.

The cell-free synthesis of proteins, i.e. outside living cells, has several advantages over cellular expression. This is all the more true if proteins are expressed that are toxic for the bacteria or non-natural amino acids shall be inserted into the proteins.

The production of lysis deficient bacteriophages in a cell-free lysate may also be carried out without any bacteriophage host bacteria, which makes it possible to produce them locally, e.g. in a laboratory without biosafety-level. At the same time, the amount of toxic by-products that are produced by a method according to the present invention is reduced, since lysed pathogenic host bacteria are not present. Furthermore, by producing lysis deficient bacteriophages, especially lysis deficient bacteriophages to be used in medicine, in a cell-free lysate according to the present invention, there is no need to test if the cell extract is free of toxins and other harmful substances.

Another advantage is that if the *in vitro* method is based on a cell-free lysate only the genome of the added lysis deficient virus or lysis deficient bacteriophage is applied and thus, a pure solution of lysis deficient viruses or lysis deficient bacteriophage can be produced, without any pro-viruses or pro-phages. The cell-free expression in combination with the *in vitro* reproduction of nucleic acids and in particular the genome represents a broadly applicable method that accelerates the application of bacteriophages in research and development and biomedical applications.

Thus, a lysis deficient virus, in particular a lysis deficient bacteriophage, is provided by the methods in the present invention.

A further aspect of the invention relates to an all *in vitro* method for the amplification and provision of lysis deficient viruses, in particular lysis deficient bacteriophages, i.e. fully *in vitro* generated lysis deficient viruses and/or bacteriophages. According to the present invention, a "lysis deficient phage" can be generated by introduction of nucleic acid mutations during polymerase chain reaction providing holin, endolysin and/or spanin knockout mutants by deletion, insertion and/or point mutation of the template nucleic acids.

By the present invention, it could be shown for the first time that it is possible to produce synthetic lysis deficient phages *in vitro* completely, which accelerates and facilitates the production as well as the modification of lysis deficient viruses and/or lysis deficient bacteriophages.

The concentration of lysis deficient bacteriophages generated *in vitro* is so high that the corresponding composition can directly be applied in a therapy with lysis deficient phages without a further concentration step. However, if needed, a further concentration step, in particular after isolation according to step (e) and purification, is of course also within the scope of the present invention.

A further aspect of the invention relates to a composition, in particular for the use as pharmaceutical composition, comprising at least one type of lysis deficient virus and optionally at least one further other kind of a virus or bacteriophage, in particular lysis deficient virus or bacteriophage, and suitable excipients. Thus, such composition may comprise different lysis deficient bacteriophage strains, i.e. lysis deficient phage mixtures.

In addition, such composition may comprise a pharmaceutically acceptable carrier.

"Pharmaceutically acceptable carrier" relates to pharmaceutically-acceptable, fillers or diluents used to formulate pharmaceutical compositions for animal or human administration. The pharmaceutical compositions may further comprise pharmaceutically acceptable auxiliary agents, and optionally other therapeutic agents. In particular for oral administration it is preferred to add an antacid, i.e. a substance which neutralizes stomach acidity, thereby increasing the number of phages surviving passage through the stomach.

The pharmaceutical compositions may be used in combination with antibiotics for the purpose of treating bacterial infections and/or to treat antibiotic resistant bacteria. In particular, bacteriophages tend to be more successful than antibiotics where there is a biofilm covered by a polysaccharide layer, which antibiotics typically cannot penetrate. According to a preferred embodiment, the composition may be freeze dried.

The dose and regimen of administration of a pharmaceutical composition will necessarily be dependent upon the therapeutic effect to be achieved (e.g. treatment of IBD) and may vary with the particular lysis deficient bacteriophage strains in the composition, the route of administration, and the age and condition of the individual subject to whom the pharmaceutical composition is to be administered.

If the pharmaceutical composition is to be administered with one or more antibiotics, it may be simultaneously, separately or sequentially administered.

Pharmaceutical compositions and routes of administration include those suitable for or via oral (including buccal, sublingual and intraorbital), rectal, nasal, topical (including transdermal), ocular, vaginal, bronchial, pulmonary or parenteral (including subcutaneous, intramuscular, intravenous, intradermal, intraperitoneal, intrapleural, intravesicular and intrathecal) administration or administration via an implant.

In particular a freeze-dried composition may be administered orally, preferably in form of a pill.

A further aspect of the invention relates to the use of such composition or lysis deficient virus, in particular lysis deficient bacteriophage provided as described herein for use in medicine, chemistry, biotechnology, agriculture and/or food industry. Such uses are not limited to bacteria related fields and comprise, for example, the use as vaccine, for example against bacterial infections as well as tumors, and the use as a delivery vehicle for any kind of drug, in particular anti-cancer drugs, to a target cell, in particular a human target cell.

The use in bacteria related fields is, however, preferred. Lysis deficient bacteriophages may in particular be used as bacterial population control alternatives to antibiotics. They are much more specific than antibiotics and are typically harmless not only to the host organism but also to other beneficial bacteria, such as the gut mi-crobiota, as they are very selective in the strains of bacteria they are effective against. Thereby the chances of opportunistic infections are significantly reduced. Advantages include further reduced side-effects and reduced risk of the bacterium's developing resistance. Because phages replicate *in vivo,* small effective doses can be used.

These unique properties make them highly promising antimicrobials.

Agriculture comprises plant agriculture as well as animal agriculture, wherein lysis deficient bacteriophages can replace or complement antibiotics and/or pesticides.

Bacterial pathogens are associated with a couple of plant diseases which can also be treated with synthetic lysis deficient bacteriophages provided herein. Non limiting examples for bacterial plant diseases which might be treated are leaf blight on onion, potato scab or soft rot of potato, bacterial wilt on tobacco, citrus bacterial spot or citrus canker of citrus, fire blight on apple, black rot of cabbage, soft rot of calla lilies, bacterial wilt of tomato and bacterial spot of peach.

The use of the inventive synthetic lysis deficient bacteriophages in animal agriculture is exemplified by the use against *Salmonella,* pathogenic E. *coli, Clostridium,* and *Campylobacter* for the poultry industry and against *Salmonella* and pathogenic *E. coli* for the pig industry.

Lysis deficient phages provided herein may also be used to safe food products, and to forestall spoilage bacteria. Since 2006, the United States Food and Drug Administration (FDA) and United States Department of Agriculture (USDA) have approved several bacteriophage products. For example, LMP-102 (Intralytix) was approved for treating ready-to-eat (RTE) poultry and meat products. In that same year, the FDA approved LISTEX using bacteriophages on cheese to kill *Listeria* monocyto-genes bacteria.

With regard to biotechnology related uses, the methods described as well as the lysis deficient bacteriophages provided herein can, for example, be used for phage display related application.

The invention further includes a kit comprising a pharmaceutical composition of the invention and instructions for the use of the composition for a use as hereinbefore described, optionally together with packaging material.

Further aspects relate to the use of a virus, in particular a bacteriophage provided herein in methods for the prevention and/or treatment of a bacterial infection as well as for avoiding bacterial growth.

### Figures

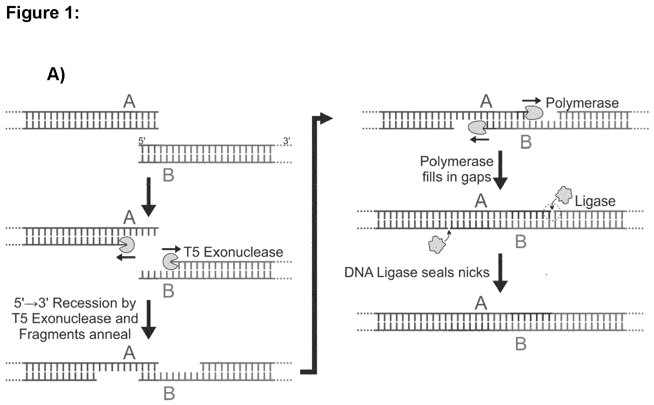

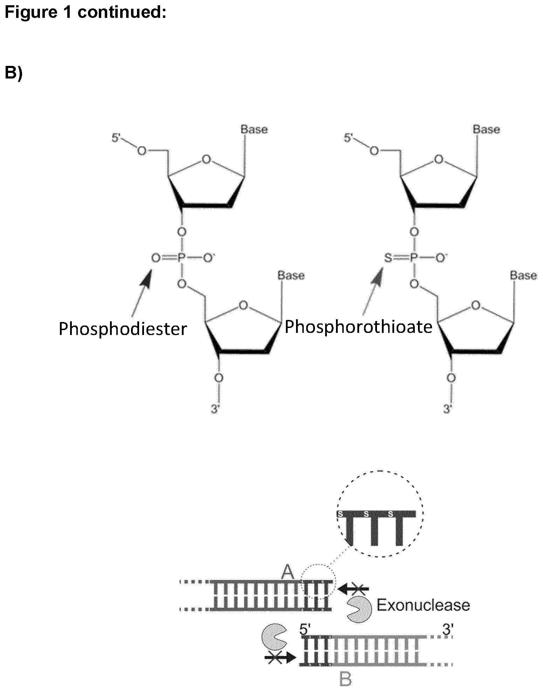

- **Figure 1:**: *In vitro* DNA manipulation (A) Gibson assembly (B) PTO (Phosphorothioate) bonds to protect from T5 exonuclease

### Example

### Producing of E.coli lysis deficient phages

The T7 *E.coli* phage has a genome that is 40 kbp in size with overlapping sequences at its ends. This genome was completely produced synthetically with the method according to the present invention.

As a first step, the template was methylated with Dam-methylase.

Subsequently, three approximately 10 kbp long pieces (10.6, 12.2, 14 kbp) and a slightly shorter one at the end are amplified in a PCR reaction. Herein, in gene 17.5 (holin) (Xu et al. 2021) and in gene 3.5 (lysine) (Inouye et al. 1973) simultaneously a knockout through a substitution was inserted. Via primer design, the start codon was deleted and additionally a frameshift was inserted. The first and the last primer at the genome were modified in that the first eight base pairs showed phosphorothioate bonds in the backbone and, unlike the other primers, only a phosphodiester backbone. In detail, the primers used at both ends of the genome had phosphorothioate bonds (in the backbone) between the first 8 bases, the remaining nucleotides were linked via (regular) phosphodiester bonds.

After the PCR reaction the methylated template DNA was removed by the enzyme Dpnl and primers in excess and the removed template were removed by a PCR-clean-up kit.

The five DNA fragments were added in a stoichiometric ratio of 1:1:1:1:1 and assembled with a Gibson mastermix.

Afterwards the DNA was added to a cell-free expression system. The cell-free expression system was *E.coli* S30 cell extract that was produced according to the protocol of Z.Z. Sun et al. (Z.Z. Sun, C.A. Hayes, J. Shin, F. Caschera, R.M. Murray, V. Noireaux, J Vis Exp. 2013, 79, 50762).

Then the lysis deficient bacteriophages that were completely produced synthetically were verified via a spot assay (inhibitory effect on growth) as well as via TEM.

Thus, this method provides a fast and easy way to produce lysis deficient phages.

## Claims

1. *In vitro* method for the provision of a lysis deficient viruses, in particular a lysis deficient bacteriophage, comprising the steps
(a) providing a virus nucleic acid, in particular bacteriophage nucleic acid, comprising a holin, endolysin and/or spanin knockout mutation,
(b) providing a cell-free expression system,
(c) combining the virus nucleic acid, in particular the bacteriophage nucleic acid, and the cell-free expression system, and
(d) incubating the combined virus nucleic acid, in particular bacteriophage nucleic acid, and the cell-free expression system under conditions suitable for expression and self-assembly of the lysis deficient virus, in particular lysis deficient bacteriophage, and
(e) isolating the self-assembled lysis deficient virus, in particular bacteriophage.

2. The method of claim 1,
wherein step (a) comprises
(a') *in vitro* amplification of a virus nucleic acid, in particular a bacteriophage nucleic acid such as a bacteriophage genome, encoding the lysis deficient virus, in particular lysis deficient bacteriophage, to be provided, preferably comprising the steps of
(i) providing a virus nucleic acid template, in particular a bacteriophage nucleic acid template,
(ii) sensitizing, in particular methylating, the virus nucleic acid template, in particular the bacteriophage nucleic acid template, for modification specific restriction enzymes,
(iii) amplification of the virus nucleic acid template, in particular the bacteriophage nucleic acid template, using polymerase chain reaction to amplify and provide different nucleic acid segments thereby introducing nucleic acid mutations to provide holin, endolysin and/or spanin knockout mutants,
wherein the primers used for at least one 5'-end of the virus nucleic acid template, in particular the bacteriophage nucleic acid template, optionally introduce nucleic acid exonuclease degradation blocking modifications, in particular phosphorothioate bonds, between the first nucleotides at the at least one 5'-end of the corresponding nucleic acid segments,
(iv) digesting the methylated virus nucleic acid template, in particular the bacteriophage nucleic acid template,
(v) recessing by an 5'-3'-directional exonuclease, in particular T5 exonuclease, to provide sticky end nucleic acid segments,
(vi) annealing of the sticky nucleic acid segments and
(vii) ligating of the annealed nucleic segments to provide amplified linear virus nucleic acids, in particular bacteriophage nucleic acids.

3. The method of claim 1 or 2,
wherein the virus nucleic acid, in particular bacteriophage nucleic acid, or virus nucleic acid template, in particular bacteriophage nucleic acid template, is selected from the group consisting of DNA, RNA and variants thereof.

4. The method of any of claims 2-3,
wherein the nucleic acid mutations providing holin, endolysin and/or spanin knockout mutants are introduced by deletion, insertion and or point mutation of the template nucleic acids.

5. The method of any of claims 2-4,
wherein the nucleic acid mutations provide holin and endolysin knockout mutants.

6. The method of any of claims 2-5,
wherein the sensitizing step (ii) is performed by
the use of Dam-methylase and/or digested by the use of Dpnl.

7. The method of any of claims 1-6,
wherein the cell-free expression system is host independent and preferably selected from cell lysates or artificial expression systems.

8. The method of any of claims 1-7,
wherein the cell lysates are derived from microorganisms, in particular E. *coli,* yeast, insects, mammals, plants and/or are artificial.

9. The method of any of claims 1-8,
wherein at least one protein and/or a nucleic acid encoding a protein to enhance or otherwise improve the expression and/or self-assembly, preferably selected from the group comprising co-factors, chaperons, polymerases, transcription regulatory factors, and/or any mixtures thereof, is added during step (b), (c) and/or (d).

10. Lysis deficient virus, in particular lysis deficient bacteriophage, provided by the method of any of claims 1-9.

11. An all *in vitro* method for the amplification and provision of lysis deficient synthetic bacteriophages.

12. Composition, in particular pharmaceutical composition, comprising at least one type of lysis deficient virus, in particular lysis deficient bacteriophage according to claim 10 and optionally at least one further other kind of a virus or bacteriophage, in particular lysis deficient virus or lysis deficient bacteriophage, and suitable excipients.

13. Lysis deficient virus, in particular lysis deficient bacteriophage, according to claim 10 or composition according to claim 12 for use in medicine, chemistry, biotechnology, agriculture and/or food industry.

14. Lysis deficient virus, in particular lysis deficient bacteriophage, of according to claim 10 or composition according to claim 12 for use in a method for the prevention and or treatment of a bacterial infection.

15. Use of a lysis deficient virus, in particular a lysis deficient bacteriophage, according to claim 13 or 14 for avoiding bacterial growth.
